# EUROPEAN PATENT APPLICATION

(11) **EP 4 530 007 A1**
(43) Date of publication of application: **02.04.2025**
(21) Application number: 24192653.4
(22) Date of filing: 02.08.2024
(51) Int. Cl.: B23K 9/32, A61F 9/06

(54) **WELDING INFORMATION PROVIDING APPARATUS**

(30) Priority: 27.09.2023 KR 20230130702
(71) Applicant: Otos Wing Co., Ltd., Seoul 08521 (KR)
(72) Inventor: HUH, Moon Young, 08521 Seoul (KR); HUH, Sung Won, 06084 Seoul (KR)
(74) Representative: HGF

(57) **Abstract**

The present disclosure relates to a welding information providing apparatus.

The welding information providing apparatus includes a body provided to be wearable by a user, a camera unit including a first camera mounted on the body and configured to image a welding site in which welding work is not in progress to acquire a video frame and a second camera mounted in a position different from the first camera and configured to image a welding site in which welding work is in progress to acquire a video frame, a video generating unit configured to generate and provide a processed image based on the video frame acquired by the first camera or the second camera, and a display unit configured to display the processed image provided from the video generating unit to the user.

## Description

### BACKGROUND

### 1. Field

The present disclosure relates to a welding information providing apparatus, and more particularly, to a welding information providing apparatus capable of protecting users' eyes by using a plurality of cameras and providing images from the same position even when different cameras are used.

### 2. Description of the Related Art

Protective gear, such as welding masks, have been used to protect users from light and high heat occurring during a welding process. While wearing protective gear, users may only identify the progress of welding through the protective gear, and thus, in order to check various information for welding, such as conditions set for welding devices, users have to inconveniently remove the protective gear and identify the information with naked eyes.

When users are not skillful, in particular, when users wear an automatic welding face and a manual welding face, users may view only a portion adjacent to welding light and it may be difficult to recognize a specific welding situation, such as an environment around the welding. Therefore, there is a need to provide users with high-definition images that allow them to visually check even an environment surrounding the welding and to provide users with specific information on welding status information.

Moreover, during welding work, the illuminance/brightness of a welding light spot is very high, so a blackening filter is used to protect the user's eyes from the welding light spot and to ensure smooth welding work. In this case, regions other than the welding light spot are completely invisible, making welding work very difficult, and the accuracy of welding may rather deteriorate.

The above problems may cause the same problems to medical staff not only during welding work, but also during skin procedures and/or treatment using high-brightness/high-illuminance light, such as laser light, and the same problem may arise in work using other high-brightness/high-illuminance light.

### SUMMARY

The present disclosure provides a welding information providing apparatus capable of protecting users' eyes by imaging a welding site using different cameras respectively before, during, and after a welding work.

The present disclosure also provides a welding information providing apparatus capable of providing images from the same position to a user even if a specific position is imaged using different cameras.

However, these problems are illustrative, and the problems to be solved by the present disclosure are not limited thereto.

Additional aspects will be set forth in part in the description which follows and, in part, will be apparent from the description, or may be learned by practice of the presented embodiments of the disclosure.

A welding information providing apparatus including a body provided to be wearable by a user, a camera unit including a first camera mounted on the body and configured to image a welding site in which welding work is not in progress to acquire a video frame and a second camera mounted in a position different from the first camera and configured to image a welding site in which welding work is in progress to acquire a video frame, a video generating unit configured to generate and provide a processed image based on the video frame acquired by the first camera or the second camera, and a display unit configured to display the processed image provided from the video generating unit to the user.

In addition, the first camera and the second camera may be driven in a switching manner.

In addition, the camera unit may further include a frame fixing the first camera and the second camera and mounting the first camera and the second camera on the body.

In addition, the camera unit may further include a light blocking cartridge coupled to the frame and located in front of the second camera.

In addition, the light blocking cartridge may include a liquid crystal panel configured to block welding light.

In addition, the first camera and the second camera may be arranged to face in directions that are not parallel to each other.

In addition, the welding information providing apparatus may further include a path changing unit configured to guide the processed image provided from the video generating unit to the display unit.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a diagram illustrating a structure of a welding system according to an embodiment of the present disclosure;
FIG. 2 is a block diagram illustrating components of the welding information providing apparatus of FIG. 1;
FIG. 3 is a perspective view of a welding information providing apparatus according to an embodiment of the present disclosure;
FIG. 4 is a rear view of the welding information providing apparatus of FIG. 3;
FIG. 5 is a cross-sectional view taken along line I-I' of FIG. 3;
FIG. 6 is an enlarged view of portion A of FIG. 5;
FIG. 7 is a diagram illustrating an operation of a camera module including a plurality of cameras;
FIG. 8 is a diagram schematically illustrating an image captured through the camera module of FIG. 7; and
FIGS. 9 and 10 are diagrams illustrating a camera unit according to an embodiment of the present disclosure.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions, such as "at least one of," when preceding a list of elements, modify the entire list of elements and do not modify the individual elements of the list.

As the present disclosure allows for various changes and numerous embodiments, particular embodiments are illustrated in the drawings and described in detail in the written description. The advantages and features of the embodiments and methods of achieving them will be apparent from the following embodiments that will be described in more detail with reference to the accompanying drawings. However, the present disclosure is not limited to the embodiments disclosed below and may be implemented in various forms.

Hereinafter, embodiments of the present disclosure are described in detail with reference to the accompanying drawings. When describing with reference to the drawings, identical or corresponding components will be assigned the same reference numerals and redundant description thereof will be omitted.

In the following embodiments, terms, such as "first," "second," etc. are used not in a limiting sense but for the purpose of distinguishing one component from another component.

In the following examples, singular terms include plural terms unless the context clearly dictates otherwise.

In the following embodiments, the terms such as "including" "having," and "comprising" are intended to indicate the existence of the features or components disclosed in the specification, and are not intended to preclude the possibility that one or more other features or components may exist or may be added.

In the drawings, the sizes of components may be exaggerated or reduced for convenience of explanation. For example, the size and thickness of each component shown in the drawings are shown arbitrarily for convenience of explanation, so the present disclosure is not necessarily limited thereto.

In the following embodiments, x-axis, y-axis, and z-axis are not limited to three axes on an orthogonal coordinate system, and may be interpreted in a broader sense. For example, the x-axis, y-axis, and z-axis may be orthogonal to each other, but may also refer to different directions that are not orthogonal to each other.

In cases in which an embodiment may be implemented differently, a specific process sequence may be performed differently from the described sequence. For example, two processes described in succession may be performed substantially at the same time or may be performed in an order opposite to the described order.

Hereinafter, based on the principles described above, a welding information providing apparatus 100 according to the present disclosure is described in detail with reference to the drawings.

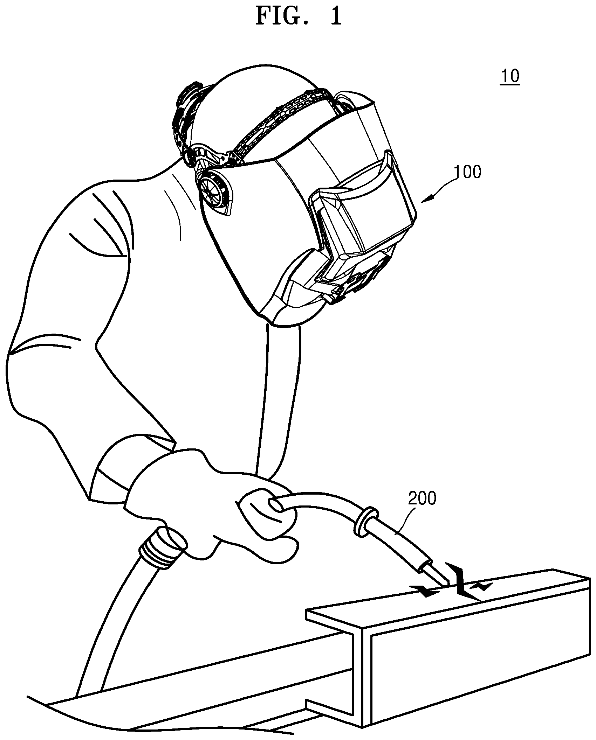

FIG. 1 is a diagram illustrating a structure of a welding system 10 according to an embodiment of the present disclosure.

Referring to FIG. 1, the welding system 10 of the present disclosure may include a welding information providing apparatus 100 and a welding torch 200. The welding information providing apparatus 100 may be connected to the welding torch 200 through a communication network and may transmit and receive data. The welding information providing apparatus 100 and the welding torch 200 may operate in a one-to-one manner, but are not limited thereto and may also operate in a one-to-n manner. In other words, n welding torches 200 may be connected to one welding information providing apparatus 100, and one welding torch 200 may be connected to n welding information providing apparatuses 100. In addition, the welding information providing apparatus 100 and the welding torch 200 may communicate with a separate server (not shown) to exchange data.

The welding information providing apparatus 100 may provide information on a welding situation to a user. In detail, the welding information providing apparatus 100 may acquire a video frame using at least one camera included in the camera unit of the welding information providing apparatus 100, generate a processed video based on the video frame, and display the generated video frame to the user. The welding information providing apparatus 100 may generate the processed video (e.g., video synthesis) using high dynamic range (HDR) technology and display and provide a high-definition processed video to the user. Here, the user may visually check a shape of a welding bead and information on the surrounding environment other than a portion adjacent to welding light through the high-definition processed video.

In the welding information providing apparatus 100 according to an embodiment of the present disclosure, a video frame may be acquired by placing a camera unit at a position equivalent to the user's field of view. Through this, the welding information providing apparatus 100 according to an embodiment of the present disclosure may acquire a video similar to that when the user directly looks at a work site and provide more accurate welding information to the user.

In addition, the welding information providing apparatus 100 according to an embodiment of the present disclosure may synthesize high-definition welding videos and provide a synthesized high-definition welding video, and to this end, videos may be acquired through two or more cameras and each video may be displayed through at least one display unit. For example, the camera unit may include two cameras, which are described in detail later.

The welding information providing apparatus 100 may repeatedly capture videos repeatedly with different shutter speeds, ISO sensitivity, and gain values of each camera and synthesize the captured videos. The welding information providing apparatus 100 according to an embodiment of the present disclosure may improve image quality by performing contrast processing on the processed video.

In addition, the welding information providing apparatus 100 of the present disclosure may provide a function of displaying welding information in a preferred color (e.g., green, blue) using RGB. In addition, the welding information providing apparatus 100 of the present disclosure may provide a magnifying glass power correction function (e.g., screen enlargement and reduction). In addition, the welding information providing apparatus 100 of the present disclosure may provide a temperature synthesis video using a separate thermal imaging camera. Here, the welding information providing apparatus 100 may display a welding temperature in color. The welding information providing apparatus 100 of the present disclosure may support a function of providing all of the aforementioned functions with sound (e.g., guidance alarm) or guidance voice.

The welding torch 200 according to an embodiment of the present disclosure may detect a welding situation including a welding temperature, a welding direction, a welding slope, a welding speed for real-time welding work and a distance between a base material and the welding torch 200 through at least one sensor. The welding torch 200 may monitor a state of a torch and change a set value of torch work depending on the welding situation.

The welding information providing apparatus 100 of the present disclosure may receive information on work settings and work status from the welding torch 200 through a communication network connected to the welding torch 200 and provide work information to the user based on the received welding information via visual feedback.

For example, when sensing information on a welding temperature value is received, the welding information providing apparatus 100 may output a notification corresponding to the temperature value in various ways, such as light, vibration, or a message. Here, the notification may be visual feedback provided on a display unit of the welding information providing apparatus 100 or may be auditory feedback through a sound (e.g., a guidance alarm) or a guidance voice.

Meanwhile, sensing information on the temperature value may include information on whether the temperature value exceeds a preset temperature range. In addition, the sensing information on the temperature value may include a numerical value, grade, level, etc. corresponding to the temperature value of a welding face.

If the temperature values of the torch and the welding face are determined to be outside the preset temperature range, the welding information providing apparatus 100 according to an embodiment of the present disclosure may guide the user to stop work. In the case of welding outside the preset temperature range, there may be a risk of quality deterioration, so the user may be guided to adjust the temperature value of the torch.

Here, the visual feedback may be providing an icon indicating danger to a partial region of the display unit of the welding information providing apparatus 100 displaying the work site. As another example, the welding information providing apparatus 100 may provide work stoppage guidance through visual feedback by repeatedly increasing and decreasing the chroma of a specific color (e.g., red) on the entire display screen.

According to an embodiment of the present disclosure, the welding information providing apparatus 100 may detect welding information through a sensor included in the welding information providing apparatus 100, in addition to at least one sensor included in the welding torch 200. Here, as the welding information, a welding situation including a level of light, a welding temperature, a welding direction, a welding slope, and a welding speed related to real-time welding work and a distance between a base material and the welding torch 200 through at least one sensor.

Similarly, based on the welding information detected through the sensor included in the welding information providing apparatus 100, the welding information providing apparatus 100 may provide guidance corresponding to the welding information.

According to an embodiment of the present disclosure, after the guidance on work stoppage is provided, the welding information providing apparatus 100 may change the operation of the welding torch 200 by sensing a preset user's movement or a preset user's voice, etc.

In another embodiment, when communication with the welding torch 200 is not smooth, the welding information providing apparatus 100 may acquire temperature values of the torch and the welding face through image sensing provided on its own. For example, the welding information providing apparatus 100 may acquire temperature values of the torch and the welding face based on image data acquired through a thermal imaging camera.

The above example only describes the case in which the information received from the welding torch 200 is welding temperature information, and of course, the welding information providing apparatus 100 may provide guidance for various welding information.

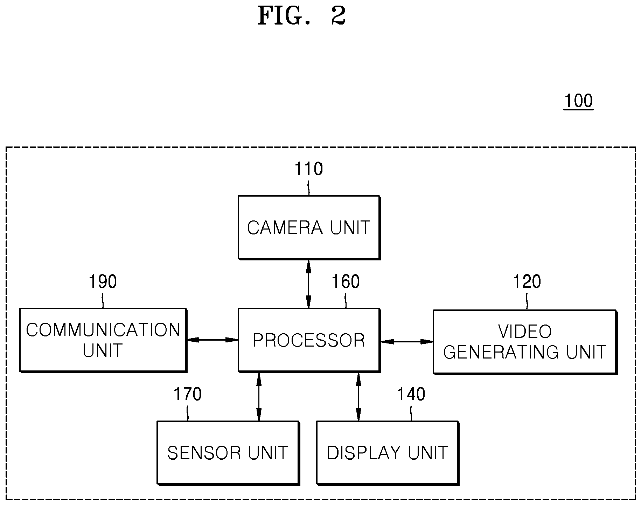

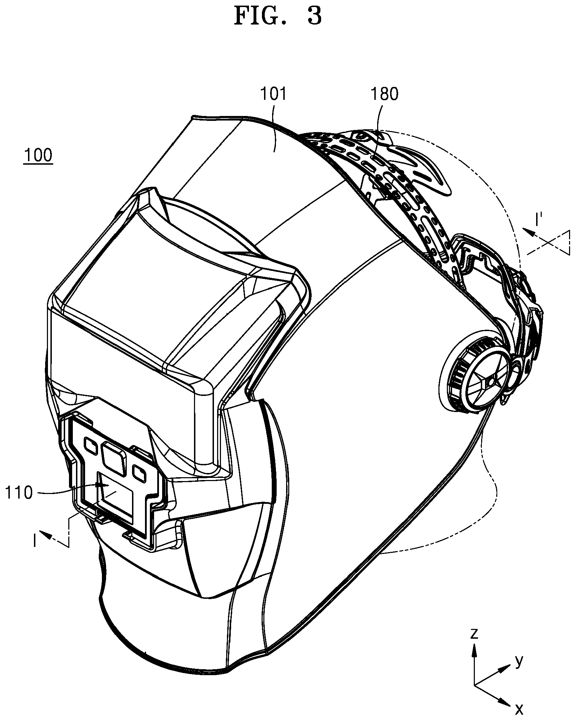

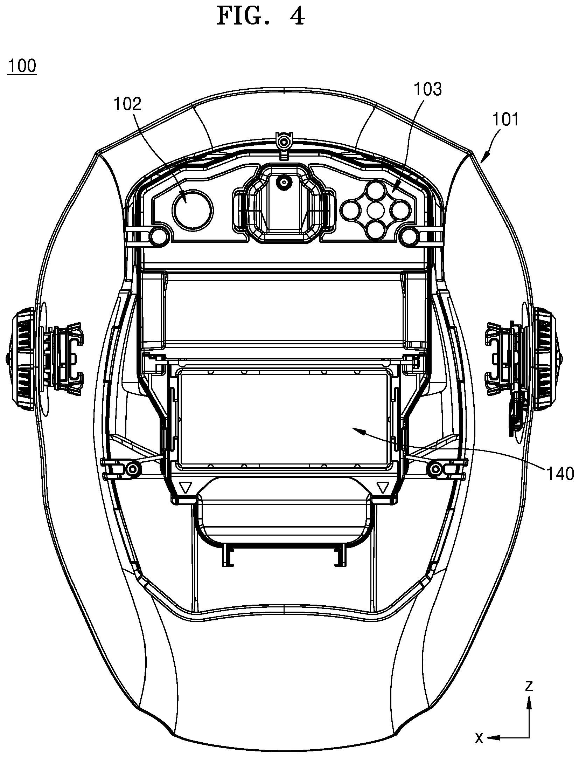

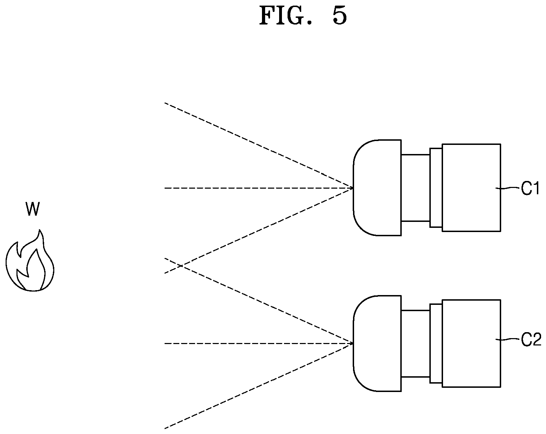

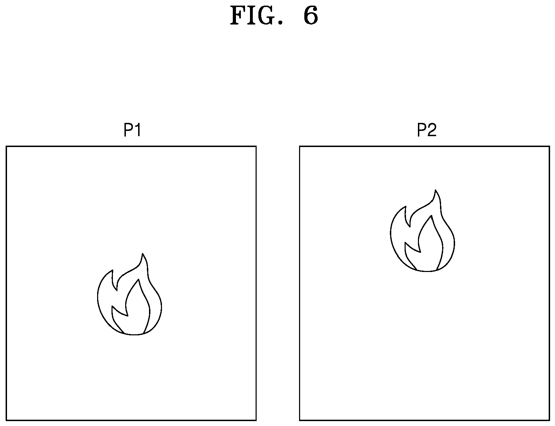

FIG. 2 is a block diagram illustrating components of the welding information providing apparatus 100 of FIG. 1, FIG. 3 is a perspective view of the welding information providing apparatus 100 according to an embodiment of the present disclosure, and FIG. 4 is a rear view of the welding information providing apparatus 100 of FIG. 3. FIG. 5 is a cross-sectional view taken along line I-I' of FIG. 3, and FIG. 6 is an enlarged view of portion A of FIG. 5.

Referring to FIGS. 2 to 6, the welding information providing apparatus 100 may include at least one of a body 101, a camera unit 110, a video generating unit 120, a path changing unit 130, a display unit 140, a lens unit 150, a processor 160, a sensor unit 170, and a communication unit 190. In addition, the welding information providing apparatus 100 may include a fixing portion 180 located on the rear of the body 101 to fix the welding information providing apparatus 100 to the user's head.

The body 101 serves to protect the user's face and may be provided so that the user may wear it. The body 101 may be formed of a material having a certain strength, such as reinforced plastic, but the present disclosure is not limited thereto, and any material that is resistant to elements, such as sparks that may occur during welding, may be used.

The body 101 may be fixed to the user's head through the fixing portion 180 provided on the inside of the body 101. The fixing portion 180 may have a structure including a plurality of ribs like a head gear, and an element including a soft material, such as a fiber material or a cushion material, may be located on at least a portion of the inner surface that is in direct contact with the worker's head.

The camera unit 110 is mounted on the outside of the body 101 and may include at least one camera 114 that acquires video frames for welding work.

The camera unit 110 may receive a control command from the processor 160 and capture a video of welding work by changing settings, such as a shutter speed, ISO sensitivity, and a gain, in response to the control command.

As an embodiment, the camera unit 110 may be located on a front portion of the body 101 corresponding to the user's field of view. The camera unit 110 may include one camera 114 and acquire a video frame for welding work, but the present disclosure is not limited thereto.

As another embodiment, the camera unit 110 may further include the camera 114 that acquires a video frame by performing imaging at a position other than the front portion of the body 101 corresponding to the user's field of view.

The camera unit 110 may include a frame 111, an outer cover plate 112, and a light blocking cartridge 113.

The frame 111 may perform a function of protecting the camera 114.

In addition, the frame 111 may perform a function of coupling the camera unit 110 to the body 101. For example, the frame 111 may perform a function of fixing at least one camera 114 included in the camera unit 110 and mounting the camera 114 on the body 101.

The outer cover plate 112 may be located in front of the light blocking cartridge 113 and may protect the light blocking cartridge 113. The outer cover plate 112 may include a material that allows light to pass therethrough, for example, a transparent material. The outer cover plate 112 may include a resin material, such as polycarbonate or acrylic, and may be molded by injection molding.

The light blocking cartridge 113 may block welding light occurring when welding occurs. That is, the light blocking cartridge 113 may be blackened based on welding light information detected through the sensor unit 170, for example, a photosensor, to increase the degree of light blocking of the cartridge.

As an embodiment, the light blocking cartridge 113 may include a liquid crystal panel 1131 (an LCD panel) whose degree of blackening may be adjusted depending on an alignment direction of liquid crystal. For example, the light blocking cartridge 113 may be implemented with various LCD panels, such as a vertical align (VA) type LCD, a twist nematic (TN) type LCD, and an in-plane switching (IPS) type LCD.

The degree of blackening of the light blocking cartridge 113 may be automatically adjusted according to the brightness of welding light. As described above, in a case in which the degree of blackening is automatically adjusted according to the brightness of welding light is automatically adjusted, the sensor unit 170 may be used. The sensor unit 170 may acquire welding light information by detecting the intensity of the welding light and transmit information on the intensity of welding light included in the welding light information as a certain electrical signal to the processor 160 to be described below. Then the processor 160 may control the degree of blackening based on the intensity of welding light.

That is, the light blocking cartridge 113 may change the degree of light blocking of the liquid crystal panel 1131 in real time to correspond to the intensity of light occurring at the welding face at welding work site, and the camera unit 110 may capture a welding video in which a certain amount of welding light is blocked by the light blocking cartridge 113 installed on the front portion.

As an embodiment, the light blocking cartridge 113 may include a coating layer 1132 located on at least one surface of the liquid crystal panel 1131. For example, the coating layer 1132 may perform an anti-reflection function.

Accordingly, the amount of welding light, that has passed through the outer cover plate 112, reflected by the coating layer 1132 is reduced, thereby minimizing or preventing a ghost phenomenon.

As an example, the coating layer 1132 may be formed by a method of forming a film by applying a coating layer, such as vacuum deposition, sputtering, and/or WET coating, and drying and/or curing the coating layer. However, the present disclosure is not limited thereto, and any coating method that may be easily selected by a person skilled in the art to which the present disclosure pertains may be employed as the method for forming the coating layer according to the present disclosure.

As an example, the coating layer 1132 may be formed and/or coated with magnesium fluoride (MgF₂), silicon (Si), and silicon dioxide (SiO₂), but is not limited to these materials.

If the reflectivity of the coating layer 1132 is too high, the amount of welding light passing through the light blocking cartridge 113 may not be sufficient, which may reduce clarity of video, and if the reflectivity of the coating layer 1132 is too high, the efficiency of the coating layer 1132 may decrease to cause a ghost phenomenon.

Accordingly, in an optional embodiment, the coating layer 1132 may be formed to have a reflectivity less than 4%, less than 3.5%, or less than 3.0%, and as another example, the coating layer 1132 may be formed to have a reflectivity of 1.0% or less.

In addition, the coating layer 1132 may be formed to have a refractive index of 1.3 to 1.36 to obtain a clear video.

As an example, the coating layer 1132 may include a single layer or multiple layers. Here, the plurality of layers may be formed to have different refractive indices.

As an example, the coating layer 1132 may be formed to have a thickness of 25 to 35 nm. If the coating layer 1132 is formed thicker than 35 nm, the weight of the welding information providing apparatus 100 may increase, which may increase the user's work fatigue, and if the coating layer 1132 is formed thinner than 25 nm, the reflectivity of the coating layer 1132 may decrease.

As an example, the camera unit 110 may include a thermal imaging camera. The welding information providing apparatus 100 may acquire a temperature video by combining a thermal video acquired through the thermal imaging camera with the image of the welding site.

As an embodiment, the welding information providing apparatus 100 may further include a lighting unit (not shown) electrically connected to the processor 160. The lighting unit (not shown),au be located outside the welding information providing apparatus 100 and may be configured to irradiate light toward at least welding work region. The lighting unit (not shown) may include a plurality of LED modules, and the level of light output through the lighting unit (not shown) may be adjusted by control of the processor 160. According to an embodiment, the lighting unit (not shown) may operate in conjunction with the operation of the camera unit 110 under control by the processor 160.

The video generating unit 120 may be located inside the body 101 and may generate a welding video based on the video frame acquired by the camera unit 110 and provide the generated welding video to the user. The video generating unit 120 may provide the welding video acquired from the camera unit 110 as is or may provide a high-definition processed video including welding information.

For example, the video generating unit 120 may provide a high-definition processed video so that the user may visually check the surrounding environment (e.g., the shape of a previously worked welding bead, etc.) other than a portion adjacent to welding light. In addition, the video generating unit 120 may provide visual feedback (e.g., a welding progress direction) with respect to a welding progress state to the user to guide welding work.

Therefore, hereinafter, a welding video may be interpreted as a meaning including both a welding video not processed yet after being obtained from the camera unit 110 and a processed video obtained by synthesizing a video frame acquired from the camera unit 110 with the aforementioned welding information.

The video generating unit 120 may include a display panel that generates light including welding video information and an optical system that diffuses light from the display panel. The video generating unit 120 may be configured as a projector capable of generating and projecting a welding video through the display panel and the optical system.

The display panel of the video generating unit 120 may generate and emit light including the welding video information. The display panel may be, but is not limited to, liquid crystal display (LCD), an organic light emitting diode (OLED), a light-emitting diode (LED), or a liquid crystal on dilicon (LCoS).

The video generating unit 120 may be located inside the body 101, but not in a position at which a welding video is directly provided to the user's field of view, but in a position at which the welding video is indirectly provided by the path changing unit 130.

As an example, the video generating unit 120 may be located so that a direction in which the welding video is provided is perpendicular to an optical axis of at least one camera 114 of the camera unit 110. In other words, as shown in FIG. 5, the video generating unit 120 may be located near the user's forehead and may provide the welding video toward the path changing unit 130.

The path changing unit 130 may change a path of the welding video provided from the video generating unit 120 and guide the welding video to the user's eyes. For example, the path changing unit 130 may include a mirror, an X-cube prism, a dichroic filter, etc. that reflect the welding video.

Accordingly, the welding information providing apparatus 100 may be configured to be small in size. For example, the video generating unit 120 may be located in various positions, such as near the user's forehead, as shown in FIG. 5, and accordingly, the size of the welding information providing apparatus 100 may be miniaturized. In other words, the welding information providing apparatus 100 has a limitation in that the size thereof should not be increased to a value or greater due to the nature that the user directly wears the welding information providing apparatus 100 and performs welding work, and this problem may be solved.

However, in the drawing, the path changing unit 130 is shown as including one mirror, but the technical idea of the present disclosure is not limited thereto, and may further include more mirrors or a member capable of changing the path to change the path of the welding video.

The welding video whose path has been changed through the path changing unit 130 may be provided to the display unit 140.

The display unit 140 may be a unit of displaying the welding video provided from the video generating unit 120 to the user. That is, the display unit 140 may allow the welding video of welding work to transmit therethrough and be provided to the user.

The display unit 140 may be located to correspond to the position of the user's eyes and may be located between the path changing unit 130 and the user's eyes to allow the welding video to transmit therethrough, thereby providing the user with the welding video. The display unit 140 may be located closer to the lens unit 150, which is to be described below, than to the path changing unit 130.

As an example, the display unit 140 may be configured as one unit. For example, the user may receive the welding video by simultaneously viewing one display unit 140 with both eyes.

As another example, the display unit 140 may be configured as two units. For example, the display unit 140 may be configured as two units respectively corresponding to the user's left and right eyes, and each display unit 140 may provide the welding video to the user's left and right eyes.

In an embodiment, the display unit 140 may be formed of a material that allows the welding video, whose path has been changed through the path changing unit 130, to transmit therethrough, so that the user may view the welding video passing through the display unit 140.

As a specific embodiment, the display unit 140 may be formed as a rear projection screen. The welding video provided from the video generating unit 120 may be projected onto the back of the screen through the path changing unit 130, and the welding video may be displayed on the surface of the screen. The welding video displayed on the surface of the screen may be provided to both eyes of the user. However, the display unit 140 is not limited thereto, and of course may be implemented with various display technologies that may allow the welding video provided from the video generating unit 120
to transmit therethrough to be provided to the user.

The welding information providing apparatus 100 may include the lens unit 150 for transferring the welding video transmitting from the display unit 140 to both eyes of the user at a short distance. The welding information providing apparatus 100 according to an embodiment of the present disclosure provides the welding video through the display unit 140 apart by a certain distance from the user. However, because it is difficult for the human eyes to focus on an object that is too close, a focal length may be adjusted by placing the lens unit 150 between the display unit 140 and the user's eyes.

That is, the lens unit 150 is located on the path along which the welding video transmitting from the display unit 140 passes, and the size of the welding video may be adjusted to be provided to both eyes of the user. By placing the lens unit 150 between the display unit 140 and the user's eyes, a distance between the user's eyes and the display unit 140 may be reduced, and the size of the welding information providing apparatus 100 may be configured to be compact. Through this, the user may secure a wide field of view and clear images even in a closed space within the welding information providing apparatus 100.

Meanwhile, the distance between the display unit 140 and the lens unit 150 may be shorter than the focal length of the lens unit 150. In addition, the lens unit 150 may project the welding video transmitting from the display unit 140 as a virtual image in front of the user. Through this, the user may feel as if the welding video is further away than an actual distance to the display unit 140, and the welding video displayed on the display unit 140 may be viewed as an enlarged virtual image, thereby obtaining the effect of viewing with a wide viewing angle.

The lens unit 150 may be formed of glass, plastic, etc., and in the case of plastic, various transparent resins, such as (meth)acrylic resin, styrene resin, and polycarbonate resin, may be used. In addition, each lens of the lens unit 150 may be coated with a filter that blocks light in a certain wavelength band. For example, the lens unit 150 may include a lens coated with a blue light blocking filter.

The processor 160 may synthesize video frames received through the camera unit 110 to generate a high-definition processed video. The processor 160 may synthesize video frames, which are acquired in time order by the camera unit 110 by setting different imaging conditions for the video frames, in parallel to acquire a processed video. In detail, the processor 160 may control the camera unit 110 to perform imaging by changing a shutter speed, ISO sensitivity, and a gain of the camera 114 of the camera unit 110.

Here, the processor 160 may set different imaging conditions depending on conditions, such as sensed welding light at a welding site, ambient light, the degree of movement of the welding torch 200, etc. In detail, the processor 160 may set the imaging conditions so that the the ISO sensitivity and gain may decrease as welding light and/or ambient light at the welding site are higher. In addition, when the movement and/or work speed of the welding torch 200 is detected to be fast, the processor 160 may set the imaging conditions so that the shutter speed increases.

The processor 160 may synthesize videos of a preset number of video frames in parallel. According to an embodiment of the present disclosure, each video within a preset video frame may be captured under different imaging conditions.

When there are two or more cameras 114 of the camera unit 110, the processor 160 according to an embodiment of the present disclosure may control each of the cameras 114 to set different imaging conditions to perform imaging. Also, in this case, the processor 160 may synthesize videos of a preset number of video frames in parallel.

In addition, the processor 160 may control the video generating unit 120 to generate a welding video based on the video frame acquired from the camera unit 110. Here, the processor 160 may control settings, such as the size, brightness, and contrast of the welding video provided to the user.

The processor 160 may control the overall operation of the welding information providing apparatus 100 using various programs stored in memory (not shown). For example, the processor 160 may include a CPU, RAM, ROM, and a system bus. Here, the ROM is a component in which an instruction set for system booting is stored, and the CPU copies an operating system stored in the memory of the welding information providing apparatus 100 to RAM according to the instructions stored in the ROM and runs the operating system to boot the system. When booting is complete, the CPU may copy various applications stored in the memory to RAM and execute the applications to perform various operations. Although the processor 160 has been described above as including only one CPU, the processor 160 may be implemented with multiple CPUs (or DSP, SoC, etc.).

According to an embodiment of the present disclosure, the processor 160 may be implemented as a digital signal processor (DSP), a microprocessor, and/or a time controller (TCON) that processes digital signals. However, without being limited thereto, the processor 160 may include one or more of a central processing unit (CPU), a micro controller unit (MCU), a micro processing unit (MPU), a controller, an application processor (AP) or a communication processor (CP), or an ARM processor or may be defined by the corresponding term. In addition, the processor 160 may be implemented as a system-on-chip (SoC) with a built-in processing algorithm or large scale integration (LSI) or may be implemented in the form of a field programmable gate array (FPGA).

The sensor unit 170 may include a plurality of sensor modules configured to detect various information on the welding site and acquire welding information. Here, the welding information may include a welding temperature, a welding direction, a welding slope, and a welding speed for real-time welding work, and a distance between a base material and the welding torch 200. Moreover, the sensor unit 170 may include an optical sensor module configured to detect a level of light within at least a welding work region.

According to an embodiment of the present disclosure, the sensor unit 170 may include an illuminance sensor, and here, the sensor unit 170 may acquire information on the intensity of welding light at the welding site. In addition to the illuminance sensor, the sensor unit 170 may further include various types of sensors, such as a proximity sensor, a noise sensor, a video sensor, an ultrasonic sensor, and an RF sensor, and may detect various changes related to a welding work environment.

The communication unit 190 is configured to receive welding information from the welding torch 200 and transmit commands for controlling the welding torch 200. According to an embodiment of the present disclosure, the communication unit 190 may transmit the processed video to an external device other than the welding torch 200. Here, the external device may include various devices including communication modules, such as a smartphone, a computer, etc. of a worker/third party.

The communication unit 190 may be configured to communicate with various types of external devices according to various types of communication methods. The communication unit 190 may include at least one of a Wi-Fi chip, a Bluetooth chip, a wireless communication chip, and a near field communication (NFC) chip. In particular, in the case of using the Wi-Fi chip or the Bluetooth chip, various connection information, such as an SSID and session key may be first transmitted and received, communication is connected using the connection information, and then, various information may be transmitted and received. The wireless communication chip refers to a chip that performs communication according to various communication standards, such as IEEE, Zigbee, 3rd generation (3G), 3rd generation partnership project (3GPP), and long term evolution (LTE). The NFC chip refers to a chip that operates in the NFC method using the 13.56 MHz band among various RF-ID frequency bands, such as 135 kHz, 13.56 MHz, 433 MHz, 860 to 960 MHz, 2.45 GHz, etc.

Meanwhile, the welding torch 200 may include a separate communication unit, sensor unit, and second processor.

The communication unit transmits and receives data to and from the welding information providing apparatus 100. The communication unit may include modules capable of short-range wireless communication (e.g., Bluetooth, Wifi, Wifi-Direct) or long-range wireless communication (3G, high-speed downlink packet access (HSDPA), or LTE).

The sensor unit or the second sensor is a component included in the welding torch 200 and sensing welding conditions, such as a welding temperature, a welding speed, a welding slope, a welding direction, and a distance between a base material and the welding torch 200.

The sensor unit may detect at least one of various changes, such as a change in posture of the user holding the welding torch 200, a change in illuminance of the welding face, and a change in acceleration of the welding torch 200, and transfer a corresponding electrical signal to the second processor. That is, the sensor unit may detect a change in state based on the welding torch 200, generate a corresponding detection signal, and transmit the detection signal to the second processor.

In the present disclosure, the sensor unit may include various sensors, and when the welding torch 200 is driven (or based on user settings), power may be supplied to at least one preset sensor according to control to detect a change in state of the welding torch 200.

In this case, the sensor unit may include at least one device among all types of sensing devices that may detect a change in state of the welding torch 200. For example, the sensor unit may include at least one sensor among various sensing devices, such as an acceleration sensor, a gyro sensor, an illuminance sensor, a proximity sensor, a pressure sensor, a noise sensor, a video sensor, a gravity sensor, etc. A level of light in welding work region detected through the illuminance sensor of the welding torch 200 may be transferred to the processor 160 through the communication unit, and the processor 160 may control a lighting unit (not shown) and/or the camera unit 110 based on the level of light transferred through the illuminance sensor of the welding torch 200, without passing through the sensor unit 170 of the welding information providing apparatus 100.

Meanwhile, the acceleration sensor is a component that detects a movement of the welding torch 200. In detail, the acceleration sensor may measure dynamic forces, such as acceleration, vibration, and impact of the welding torch 200, and thus may measure the movement of the welding torch 200.

The gravity sensor is a component that detects a direction of gravity. That is, a detection result of the gravity sensor may be used together with the acceleration sensor to determine the movement of the welding torch 200. In addition, the direction in which the welding torch 200 is held may be determined through the gravity sensor.

In addition to the aforementioned types of sensors, the welding torch 200 may further include various types of sensors, such as a gyroscope sensor, a geomagnetic sensor, an ultrasonic sensor, and an RF sensor and may detect various changes related to welding work environment.

The welding information providing apparatus 100 according to an embodiment of the present disclosure may further include a power switch 102.

The power switch 102 is a portion that supplies or cuts off power to the welding information providing apparatus 100 according to the present disclosure by a user operation. For example, the power switch 102 may be configured as a position change method or a button method, but is not limited thereto.

Because the welding information providing apparatus 100 according to the present disclosure includes components that require power, such as the camera unit 110 and the video generating unit 120, the user may turn off power through the power switch 102 when the welding information providing apparatus 100 according to the present disclosure is not in use in order to prevent waste of power.

The welding information providing apparatus 100 according to an embodiment of the present disclosure may further include a user input module 103.

The user input module 103 is a portion that receives user input to control each component of the welding information providing apparatus 100 according to the present disclosure. For example, the user may control various components of the welding information providing apparatus 100 according to the present disclosure by inputting a manipulation signal to the user input module 103.

The user input module 103 may be configured by a position change method, a button method, etc., but is not limited thereto.

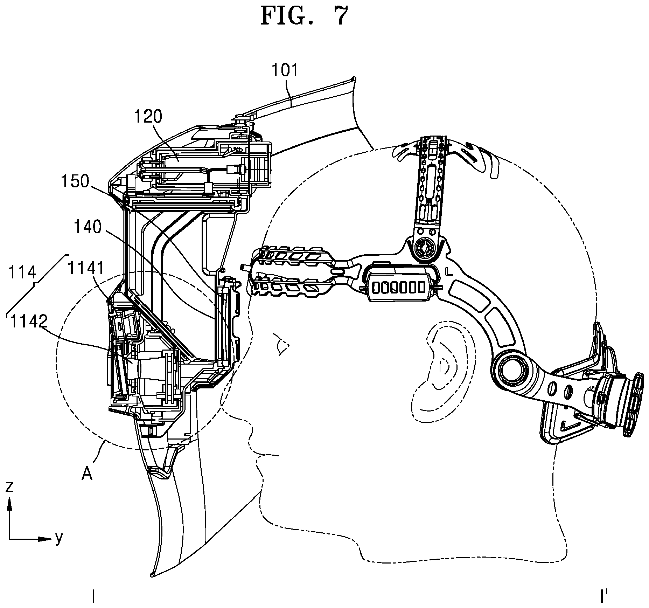

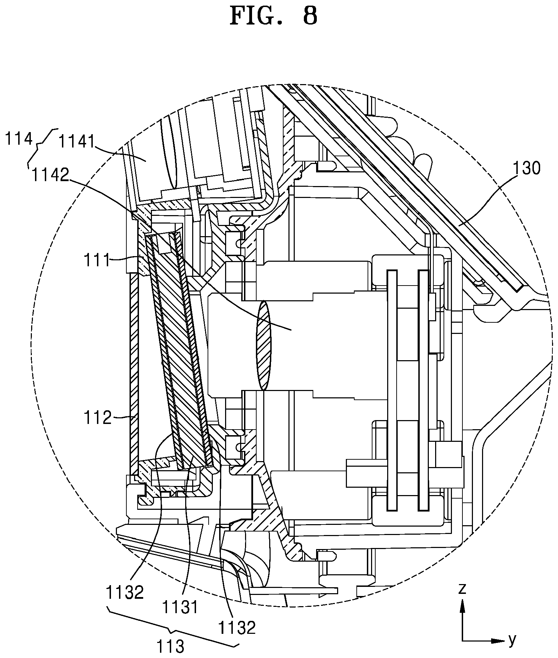

FIG. 7 is a diagram illustrating an operation of a camera module including a plurality of cameras C1 and C2, and FIG. 8 is a diagram schematically illustrating an image captured through the camera module of FIG. 7.

Referring to FIGS. 7 and 8, a camera module in which the plurality of cameras C1 and C2 are arranged side by side is shown.

Each of the camera C1 and C2 has a constant view angle range.

In this case, when the plurality of cameras C1 and C2 images a welding site W, the respective camera C1 and C2 obtain images with different upper and lower positions based on FIG. 8. For example, with reference to FIG. 7, the camera C1 located above images an upper portion of the welding site relative to the camera C2 located below. Accordingly, an image P1 captured by the camera C1 located above reflects a portion that is relatively inclined upwardly compared to the image P2 captured by the camera C2 located below.

This results from the fact that, the cameras C1 and C2 having the same view angle range are located at different positions, so the positions of the welding site W imaged by the respective cameras C1 and C2 are different by a difference in position between the cameras C1 and C2.

When this phenomenon is applied to the welding information providing apparatus 100 according to the present disclosure shown in FIGS. 3 to 5, in a case in which the camera unit 110 includes a plurality of cameras 114 and each camera 114 images the same welding site, the plurality of cameras 114 may capture positions different in a Z-axis direction.

Therefore, in a case in which the camera unit 110 includes a plurality of cameras 114 and images a certain welding site, while switching the plurality of cameras 114 according to the situation, images having different positions may be provided to the user's field of view to cause distortion or disturbance in the user's field of view.

To solve the problem, a method of imaging a wider region through a certain camera and then moving the image up, down, left, and right by a position difference from other cameras to correct the position difference may be proposed.

However, in this case, the resolution of the image may deteriorate due to reasons, such as imaging the wider region.

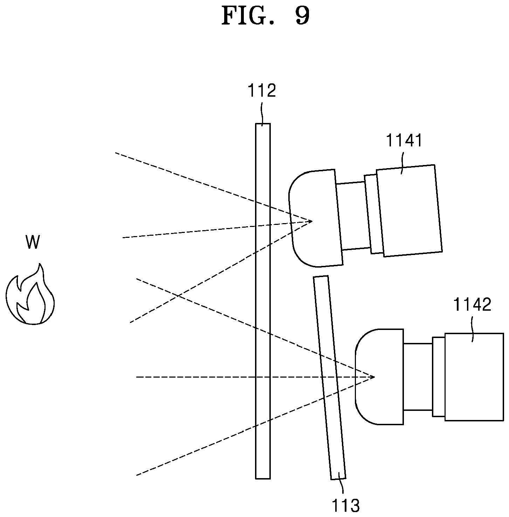

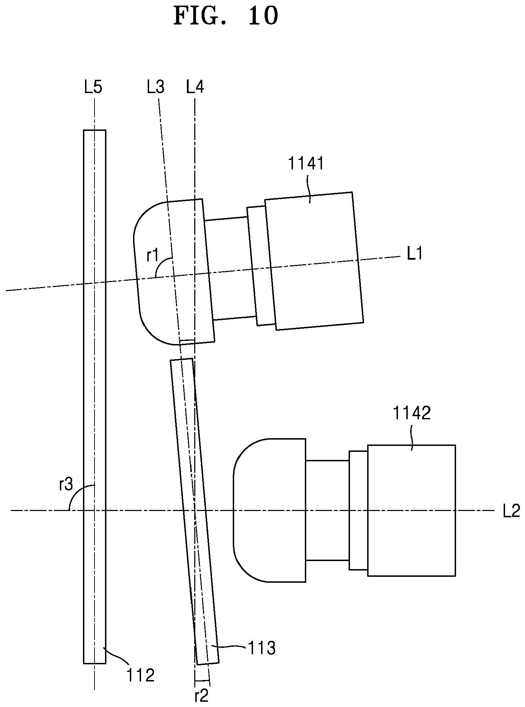

FIGS. 9 and 10 are diagrams illustrating the camera unit 110 according to an embodiment of the present disclosure.

Referring to FIGS. 5, 6, 9, and 10, the camera unit 110 according to an embodiment of the present disclosure may include a first camera 1141 and a second camera 1142.

As an example, the first camera 1141 and the second camera 1142 may be located in different positions.

As described above, the camera unit 110 may further include the frame 111. For example, the frame 111 may perform a function of fixing the first camera 1141 and the second camera 1142 and mounting the first camera 1141 and the second camera 1142 on the body 101. The frame 111 may fix the first camera 1141 and the second camera 1142 so that the first camera 1141 and the second camera 1142 are located in certain positions while forming a certain angle, as is described below.

The first camera 1141 and the second camera 1142 may be driven in a switching manner. For example, when the first camera 1141 is driven, the second camera 1142 may not be driven, and when the second camera 1142 is driven, the first camera 1141 may not be driven.

The first camera 1141 and the second camera 1142 may be selectively driven based on whether welding work is in progress.

The first camera 1141 may image the welding site W in which welding work is not in progress to acquire a video frame. For example, the first camera 1141 may be used to image the welding site W before the user starts welding work, when the user stops welding work, or when welding work is completed. That is, when welding work is not in progress, high-illuminance/high-brightness light (welding light) may not occur, so the welding site W may be imaged using the first camera 1141.

The second camera 1142 may image the welding site W in which welding work is in progress, to acquire a video frame. For example, the second camera 1142 may be used to image the welding site W when the user starts welding work. That is, when welding work is in progress, high-illuminance/high-brightness light (welding light) occurs, so the welding site W may be imaged using the second camera 1142.

As described above, the camera unit 110 may further include the light blocking cartridge 113. The configuration and function of the light blocking cartridge 113 may be the same as described above.

The light blocking cartridge 113 may be coupled to the frame 111. In addition, the light blocking cartridge 113 may be located in front of the second camera 1142.

Accordingly, a certain amount of high-illuminance/high-brightness welding light occurring by welding may be blocked by the light blocking cartridge 113. In addition, the second camera 1142 may capture a welding video in which a certain amount of welding light is blocked by the light blocking cartridge 113 installed on the front.

In addition, the light blocking cartridge 113 may not be located in front of the first camera 1141. As described above, the first camera 1141 images the welding site W while welding work is not in progress, and therefore may image the welding site W without blocking external light.

As described above, the camera unit 110 may include the outer cover plate 112. The configuration and function of the outer cover plate 112 may be the same as described above.

The outer cover plate 112 may be coupled to the frame 111. In addition, the outer cover plate 112 may be located in front of the first camera 1141 and the second camera 1142.

As an example, the outer cover plate 112 may be formed to have an area large enough to cover both the front surface of the first camera 1141 and the front surface of the second camera 1142. As another example, the outer cover plate 112 may be located in front of the first camera 1141 and in front of the second camera 1142.

As an example, the first camera 1141 and the second camera 1142 may be selectively driven by user manipulation. In detail, the user may select the operation of the first camera 1141 and the second camera 1142 through the user input module 103 or a separate module. For example, the user may drive the second camera 1142 by inputting an input signal for driving the second camera 1142 to the welding information providing apparatus 100 immediately before performing welding work.

As another embodiment, the first camera 1141 and the second camera 1142 may be selectively driven depending on the external environment. In detail, when welding light occurs externally through the sensor unit 170 or a separate module, the processor 160 may stop driving the first camera 1141 and start driving the second camera 1142.

The video generating unit 120 may generate a processed video based on a video frame acquired by the first camera 1141 or the second camera 1142. As described above, the video generating unit 120 may provide the welding video acquired from the first camera 1141 or the second camera 1142 as is or may provide a high-definition processed video including welding information.

As an example, the first camera 1141 and the second camera 1142 may be arranged to face in directions that are not parallel to each other. For example, the first camera 1141 and the second camera 1142 may be arranged to face the same welding site W.

In a specific embodiment, an imaginary line L1 (hereinafter, referred to as imaginary line L1) extending in a longitudinal direction of the first camera 1141 and passing through the center of the lens of the first camera 1141 and an imaginary line L2 (hereinafter, referred to as imaginary line L2) extending in the longitudinal direction and passing through the center of the lens of the second camera 1142 may form an angle of 5° to 11°. In some embodiments, the imaginary line L1 and the imaginary line L2 may form an angle of 8°.

The angle formed by the imaginary line L1 and the imaginary line L2 may be determined by a distance between the face of the user performing welding work and the hand performing welding work, an angle between the user's hand performing welding work and the user's field of view, the size of the welding information providing apparatus 100, the size and arrangement of each module mounted on the welding information providing apparatus 100, etc. Accordingly, when the angle formed by the imaginary line L1 and the imaginary line L2 does not range from 5 to 11 degrees, images obtained by imaging different positions according to the camera 114 being driven may be provided to the user.

In a specific embodiment, the light blocking cartridge 113 and the first camera 1141 may be arranged perpendicular to each other in space. For example, an angle r1 formed by the imaginary line L1 and the imaginary line L3 (hereinafter, referred to as the imaginary line L3) extending in a height direction of the liquid crystal panel 1131 of the light blocking cartridge 113 and passing through the center of the liquid crystal panel 1131 may be 90°. Accordingly, an angle r2 formed by the imaginary line L3 and the imaginary line L4 (hereinafter, referred to as imaginary line L4) passing through the center of the liquid crystal panel 1131 and being perpendicular to the imaginary line L2 may be 5 to 11°, or 8°.

In a specific embodiment, the first camera 1141 may be located to protrude farther from the user than the second camera 1142. In addition, as shown in FIG. 10, the light blocking cartridge 113 may be formed to extend from the first camera 1141 toward the front of the second camera 1142 and may be located in front of the second camera 1142.

In a specific embodiment, the outer cover plate 112 may be located perpendicular to the second camera 1142. For example, an angle r3 formed by the imaginary line L2 and the imaginary line L5 (hereinafter, referred to as imaginary line L5) extending in the longitudinal direction of the outer cover plate 112 may be vertical. Accordingly, the imaginary line L1 and the imaginary line L5 may form an angle of 79° to 85°, or an angle of 82°.

As such, the camera unit 110 according to an embodiment of the present disclosure may include the first camera 1141 and the second camera 1142 that are arranged at an angle to each other, and accordingly, a position difference of an image based on a position difference between the first camera 1141 and the second camera 1142 may be corrected.

In addition, according to the relative positions and tilt angles of the first camera 1141, the second camera 1142, the light blocking cartridge 113, and the outer cover plate 112, the user may be provided with a view that is not distorted or disturbed even if the driven camera 114 is switched.

As such, the present disclosure has been described with reference to the embodiments shown in the drawings, but these are merely illustrative, and those skilled in the art will understand that various modifications and equivalent other embodiments may be made therefrom. Therefore, the true scope of technical protection of the present disclosure should be determined by the technical spirit of the appended patent claims.

Specific implementations described in the embodiments are examples and do not limit the scope of the embodiments in any way. For the sake of brevity of the specification, descriptions of conventional electronic components, control systems, software, and other functional aspects of the systems may be omitted. In addition, the connections or connection members of lines between components shown in the drawings represent functional connections and/or physical or circuit connections, and may be represented by various alternative or additional functional connections, physical connections, or circuit connections in actual devices. Moreover, no component is essential to the practice of the present disclosure, unless the component is specifically described as "essential" or "critical."

In the specification of the embodiment (particularly in the claims), the use of the terms "a" and "an" and "the" and similar referents may be construed to cover both the singular and the plural. Furthermore, recitation of ranges of values herein are merely intended to serve as a shorthand method of referring individually to each separate value falling within the range, unless otherwise indicated herein, and each separate value is incorporated into the specification as if it were individually recited herein. Lastly, the steps of all methods described herein may be performed in any suitable order unless otherwise indicated herein or otherwise clearly contradicted by context. The embodiments are not necessarily limited by the order of description of the steps above. The use of any and all examples, or exemplary language (e.g., "such as") provided herein is intended merely to better describe the present disclosure and the scope of the embodiments is not limited by the examples or illustrative terms, unless limited by the claims. In addition, those skilled in the art may recognize that various modifications, combinations and changes may be made depending on design conditions and factors within the scope of the appended claims or their equivalents.

The present disclosure may provide the welding information providing apparatus capable of protecting users' eyes by imaging a welding site using different cameras respectively before, during, and after a welding work.

In addition, the present disclosure may provide the welding information providing apparatus capable of providing images from the same position to a user even if a specific position is imaged using different cameras.

However, this effect is illustrative and the effect of the present disclosure is not limited thereto.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A welding information providing apparatus comprising:
a body provided to be wearable by a user;
a camera unit including a first camera mounted on the body and configured to image a welding site in which welding work is not in progress to acquire a video frame and a second camera mounted in a position different from the first camera and configured to image a welding site in which welding work is in progress to acquire a video frame;
a video generating unit configured to generate and provide a processed image based on the video frame acquired by the first camera or the second camera; and
a display unit configured to display the processed image provided from the video generating unit to the user.

2. The welding information providing apparatus of claim 1, wherein the first camera and the second camera are driven in a switching manner.

3. The welding information providing apparatus of claim 1 or 2, wherein the camera unit further includes a frame fixing the first camera and the second camera and mounting the first camera and the second camera on the body.

4. The welding information providing apparatus of any of the claims 1 to 3, wherein the camera unit further includes a light blocking cartridge coupled to the frame and located in front of the second camera.

5. The welding information providing apparatus of claim 4, wherein
the light blocking cartridge includes a liquid crystal panel configured to block welding light.

6. The welding information providing apparatus of any of the claims 1 to 5, wherein
the first camera and the second camera are arranged to face in directions that are not parallel to each other.

7. The welding information providing apparatus of any of the claims 1 to 6, further comprising:
a path changing unit configured to guide the processed image provided from the video generating unit to the display unit.
